(19) Europäisches Patentamt
European Patent Office
Office européen des brevets

(11) Publication number: **0 081 878**
**A2**

(12) # EUROPEAN PATENT APPLICATION

(21) Application number: **82201550.9**

(22) Date of filing: **06.12.82**

(51) Int. Cl.³: **C 12 N 9/04**, C 12 R 1/06

(30) Priority: **11.12.81 IT 2551581**

(43) Date of publication of application: **22.06.83**
**Bulletin 83/25**

(84) Designated Contracting States: **AT BE CH DE FR GB LI LU NL SE**

(71) Applicant: **ANIC S.p.A., Via Ruggero Settimo, 55, I-90139 Palermo (IT)**

(72) Inventor: **Zaffaroni, Pasquale, Via Reatina 113, I-00013 Mentana (Rome) (IT)**
Inventor: **Lucchese, Giuseppe, Via F.lli Bandiera 23, I-00015 Monterontondo (Rome) (IT)**
Inventor: **Gubbiotti, Angelo, Via Galvani 20, I-05100 Terni (IT)**

(74) Representative: **Roggero, Sergio et al, Ing. Barzanò & Zanardo Milano S.p.A. Via Borgonuovo 10, I-20121 Milano (IT)**

(54) **Method for producing cells containing cholesterol oxidase.**

(57) Cells containing the enzyme cholesterol oxidase are produced by cultivating under suitable conditions a microbe strain of the Arthrobacter genus, filed in Peoria under number NRRL B-11,115.

The carbon sources used are n-paraffins and cholesterol.

EP 0 081 878 A2

This invention relates to a method for producing cells containing the enzyme cholesterol oxidase, using a strain of the Arthrobacter genus.

The use of enzymes in the analysis of complex mixtures leads to considerable precision because of the specificity shown by these biological catalysts relative to common chemical reagents. Among others, the determination of cholesterol in biological fluids has assumed considerable importance in clinical chemistry. It has been proposed to use the enzyme cholesterol oxidase for this purpose (see for example British Patent No. 1,385,320).

As stated, the object of this invention is the preparation of cells containing the enzyme cholesterol oxidase by using a microbe strain for which the ability to produce this enzyme has never been described.

This strain, deposited at the collection of the Northern Regional Research Laboratory, Peoria, Illinois, USA, under No. NRRL B-11,115, was cited in the U.K. Patent N°1.577.425 granted on January 14,1981, which related to a method for modifying the steroids based on their co-oxidation with hydrocarbons. This strain was mentioned among others, all of which were able to induce modification reactions of the initial substrate, among which it was possible to identify oxidation of cholesterol to cholestenone.

However, an examination of the contents of the aforesaid application suggested nothing regarding the possibility of

isolating a single enzymatic activity, as any one strain could be used to produce various modifications of the same substrate.

We have now discovered that which constitutes the subject matter of the present patent application, namely that it is possible to produce and isolate with high efficiency the enzyme cholesterol oxidase starting from the aforesaid microbe strain.

This strain is a non-sporogenic strictly aerobic bacterium which during a growth period of 24 hours on solid culture media passes from a coccoid form of diameter 0.7-1.0 μm to an irregular elongated form with cells having a width of 0.7-1 μm and a length of 1.5-4 μm, sometimes joined in V shape, to then again become completely coccoid. The coccoid form is clearly Gram +. During the growth cycle, the appearance of globose forms (Cystites) of a diameter of about 2-3 μm was noted.

The colonies on Nutrient Agar are opaque, with a complete periphery, and a colour which from white becomes intense pink after 3-4 days. However, on PS culture medium (see example 1) they assume an intense orange colour. The total absence of aerial mycelium is noted on both culture media.

These morphological characteristics enable the strain to be classed in the Arthrobacter genus, but it differs from that species of this genus described in Bergey's Manual of Determinative Bacteriology 8th ed. (1974) by virtue of the strong pigmentation of the colonies.

The physiological characteristics of the strain together with those reported in the literature for three strains taxonomically close to ours and described as producers of cholesterol oxidase are given in Table 1. The differences between these strains and our strain are indicated by an asterisk (*).

TABLE 1

| | Brevibacterium sterolicum | Nocardia sp. | | Arthrobacter sp. |
|---|---|---|---|---|
| | ATCC 21387[1] | NCIB 10554[2] | NCIB 10555[2] | NRRL - 11,115 |
| Gram | + | + | + | + |
| Acid fastness | ? | ? | ? | - |
| Dimensions | Rods 1.2-2.1x1.5-2.0 µm | | | Rods 0.7-1.0x1.5-4 µm, and cocci 0.7-1.0 µm |
| Obligate aerobe | No(*) | Yes | Yes | Yes |
| Spores | - | - | - | - |
| Optimum growth temperature | 37°(*) | 30° | 30° | 30° |
| Colonies on nutrient agar | Complete, shiny(*) red-orange(*) | Complete, opaque orange-creamy (*) | Complete, mucoid(*) white-creamy(*) | Complete, opaque intense pink |
| Starch hydrolysis | - | - | - | - |
| Indole | - | - | - | - |
| Urease | - | +(*) | ? | - |
| Phenylalanine determination | ? | - | - | - |
| Catalase | + | + | + | + |
| Cellulase | ? | ? | ? | - |

TABLE 1 (Continued)

| | | | | |
|---|---|---|---|---|
| $H_2S$ production | + | ? | ? | + |
| Nitrate Reduction | + | ? | ? | + |
| Gelatin liquefaction | - | +(*) | +(*) | - |
| Casein | ? | ? | ? | + |
| Oxidase (Kovacs) | ? | - | - | - |
| Voges-Proskauer test | ? | - | - | - |
| Methyl red test | ? | - | - | - |
| Acid production from sugars | ? | - | - | - |
| Compounds utilised as sole C source | | | | |
| Glucose | + | + | -(*) | + |
| Fructose | + | + | -(*) | + |
| Mannose | + | ? | ? | + |
| Galactose | + | ? | ? | + |
| Saccharose | + | + | -(*) | + |
| Maltose | +(*) | +(*) | +(*) | - |

TABLE 1 (Continued)

| | | | | |
|---|---|---|---|---|
| Lactose | -(*) | -(*) | ? | + |
| Mannitol | - | - | - | - |
| Xylose | + | ? | + | + |
| Arabinose | +(*) | - | +(*) | - |
| Cholesterol | + | ? | ? | + |
| n-hexadecane | ? | ? | ? | + |
| Lactate | ? | + | + | + |
| Oleate | ? | ? | ? | + |

1. T. UWAJIMA, H. YAGI, S. NAKAMURA and O. TERADA - Agr. Biol. Chem. 37, 2345-2350 (1973).

2. W. RICHMOND - Br. Patent 1,385,319 26 Feb. 1975.

The microbe strain described heretofore is characterised by its capacity to use both n-paraffins and cholesterol as the sole source of C, and by the fact that the oxidation of the cholesterol in a proliferative culture is intensified by the presence of n-paraffins.

We have observed that in the presence of n-paraffins the strain produces a greater quantity of cholesterol oxidase.

We have also observed that our strain produces the enzyme cholesterol oxidase even when cholesterol is absent, but its production increases if the culture medium contains cholesterol.

The invention is illustrated but not limited by the following examples.

EXAMPLE 1

Effect of n-paraffins.

The film grown for 24-48 hours at 30° in a Roux bottle containing PS culture medium of the following composition:

| | |
|---|---|
| Peptone | 6 g/l |
| Yeast autolysate | 3 " |
| Hydrolysed casein | 4 " |
| Meat extract | 1.5 " |
| Glucose | 5 " |
| Agar | 25 " |

is washed with 25 ml of sterile $H_2O$, and two 2 litre flat bottomed flasks are innoculated with 5 ml of the suspension,

the flasks containing 800 ml of preculture medium of the following composition:

| | | |
|---|---|---|
| $K_2HPO_4$ | 2 | g/l |
| $MgSO_4.7H_2O$ | 0.3 | " |
| $CaCl_2$ | 0.2 | " |
| $(NH_4)_2SO_4$ | 2 | " |
| Corn Steep Liquor (CSL) | 20 | " |
| Enzymatic hydrolysed casein | 10 | " |
| Glucose | 10 | " |

pH 7.0 sterilised at 110°/40 min.

The flasks were incubated for 24 hours at 30° on a rotary shaker (220 rpm, 3 cm movement).

10% of the precultures were used to innoculate 7.5 litre jars containing 4.5 litres of CO 15/1 and CO 38 culture medium respectively, of the following composition:

| | CO 15/1 | CO 38 | |
|---|---|---|---|
| $K_2HPO_4$ | 2 | 2 | g/l |
| $MgSO_4.7H_2O$ | 0.3 | 0.3 | " |
| $CaCl_2$ | 0.2 | 0.2 | " |
| Glucose | 20 | - | " |
| CSL (treated[1]) | 10 | 10 | " |
| Vegedor[2] | 10 | 10 | " |
| Cholesterol | 0.5 | 0.5 | " |
| n-paraffins[3] | - | 10 | ml/l |

1. The quantity of Corn Steep Liquor (CSL) is diluted with two volumes of $H_2O$, the pH is adjusted to 7.0 with NaOH, and the solution is then heated to 100° and kept boiling for

ten minutes. It is filtered while hot after adding 3% w/v of celite.

2. Vegetable protein concentrate produced by Brook Bond Liebig Italiana S.p.A.

3. A mixture of $C_{13}$-$C_{21}$ n-paraffins extracted by the Molex process from hydrogenated gas oil. The paraffins were added gradually:

| Time (hours) | ml/1 |
|---|---|
| 0 | 1 |
| 6 | 1 |
| 10 | 2 |
| 14 | 3 |
| 18 | 3 |

The jar with the culture medium CO 15/1 was incubated at 30°, 300 rpm and 0.5 v/v/min. of air. That with the culture medium CO 38 was cultivated at the same temperature and aeration, but agitated at 600 rpm.

After 21 hours of fermentation, the culture broth CO 15/1 contained 0.35 U/ml of cholesterol oxidase, while the culture broth CO 38 contained 0.45 U/ml.

The enzymatic activity was determined in the culture broth as such by the following method:

1. <u>Reagents</u>

a - Phosphate buffer 0.1 M pH 6.7

Phenol 14 mM

4-amino-antipyrine 0.82 mM

The phenol and the 4-amino-antipyrine dissolve in the

phosphate buffer.

b - Cholesterol 0.5% in Triton X100 (US 4,093,517 June 6 1978)

c - Sodium azide 0.5% in $H_2O$

d - Peroxidase (Boehringer 100 U/mg) 10 mg/ml of phosphate buffer (POD).

2. Procedure

The following are placed in a glass cuvette:

- 2.6 ml of buffer with phenol and 4-amino-antipyrine (a)
- 100 µl of enzyme (e.g. culture broth as such)
- 100 µl of cholesterol in Triton X100 (b)
- 100 µl of $NaN_3$ (c)
- 100 µl of Peroxidase (d)

It is agitated, and the colour increase at 500 nm is recorded relative to a blank sample to which the peroxidase has not been added.

The reactions are:

$$\text{Cholesterol} + O_2 \xrightarrow{\text{Cholest. Ox.}} \text{Cholestenone} + H_2O_2$$

$$2H_2O_2 + \text{4-amino-antipyrine} \xrightarrow{\text{POD}} \text{quinoneimine} + 4H_2O,$$ i.e. two moles of cholestenone are produced for each mole of quinoneimine.

3. Calculation

The cholesterol oxidase activity is calculated using the following formula:

$$U/ml = \frac{\Delta\ A/min.V.2}{12.77\ .\ Q}$$

where:

V = volume of the reaction mixture in ml

12.77 = coefficient of molar extinction of quinoneimine

Q = sample volume in ml.

EXAMPLE 2

Effect of cholesterol.

An experiment was carried out as in Example 1, using only the culture medium CO 15/1. However, the cholesterol was omitted in one jar. After 32 hours of incubation, the culture broth containing cholesterol contained 0.23 U/ml of cholesterol oxidase, whereas that without cholesterol contained 0.19 U/ml of cholesterol oxidase.

CLAIM

1. A method for producing cells containing cholesterol oxidase, consisting of cultivating the Arthrobacter microbe strain NRRL-B 11,115 in a medium containing n-paraffins and/or cholesterol as the source of carbon.